# EUROPÄISCHE PATENTSCHRIFT

(11) **EP 0 528 794 B1**
(45) Veröffentlichungstag und Bekanntmachung des Hinweises auf die Patenterteilung: **05.10.1994**
(21) Anmeldenummer: 90917122.5
(22) Anmeldetag: 04.12.1990
(51) Int. Cl.: B41J 2/165

(54) **VORRICHTUNG ZUM ERZEUGEN UND ENTKOPPELN UNTERSCHIEDLICHER BEWEGUNGEN BEI REINIGUNGS- UND DICHTSTATIONEN IN TINTENDRUCKEINRICHTUNGEN**
DEVICE FOR GENERATING AND DECOUPLING DIFFERENT MOVEMENTS IN CLEANING AND SEALING STATIONS IN INK PRINTERS
DISPOSITIF POUR L'ENGENDREMENT ET LE DESACCOUPLEMENT DE DIFFERENTS MOUVEMENTS DANS LE CAS DE STATIONS DE NETTOYAGE ET D'ETANCHEITE DANS DES IMPRIMANTES A JET D'ENCRE

(30) Priorität: 09.01.1990 DE 4000417
(43) Veröffentlichungstag der Anmeldung: 03.03.1993
(73) Patentinhaber: EASTMAN KODAK COMPANY, Rochester, New York 14650 (US)
(72) Erfinder: LEHNA, Heinz, D-8200 Rosenheim (DE)
(74) Vertreter: Pohle, Reinhard, Dipl.-Phys.
(86) Internationale Anmeldenummer: DE9000939
(87) Internationale Veröffentlichungsnummer: WO9110568

(56) Entgegenhaltungen:
- EP-A- 0 037 901
- DE-A- 3 736 916

## Beschreibung

Die Erfindung bezieht sich auf eine Vorrichtung zum Erzeugen und Entkoppeln unterschiedlicher Bewegungen bei Reinigungs- und Dichtstationen in Tintendruckeinrichtungen gemäß dem Patentanspruch 1.

Tintendruckeinrichtungen rücken neben einer Vielzahl von anderen Textendgeräten, wie z. B. Nadel-, Matrix-, Thermo-Transfer- und elektrofotografischen Druckeinrichtungen, durch die Entwicklung immer leistungsfähigerer Mikroprozessoren für den Anwender immer mehr in den Mittelpunkt. Durch die zunehmende Leistungsfähigkeit der textverarbeitenden Peripheriegeräte ist die Anschaffung eines Druckers häufig eine strategische Entscheidung, bei der Leistungsmerkmale, wie Schnelligkeit, Wirtschaftlichkeit und Schriftbildqualität in den Vordergrund treten. Darüber hinaus wird das Leistungsmerkmal, koloriert drucken zu können, für verschiedene Anwendungsgebiete an Bedeutung zunehmen. Neben dem Thermo-Transferdruck bietet hierfür der Tintendruck durch die einfache und kostengünstige Herstellung von farbigen Schreibflüssigkeiten optimale Voraussetzungen. Die in einer Tintendruckeinrichtung eingesetzten Tintenfarben sind beispielsweise gemäß der DE-A1-37 36 916 gelb, cyan, magenta und schwarz. Den Vorteil, den die Tintendruckeinrichtungen bei der kostengünstigen und einfachen Herstellung der Tinte besitzen, steht der Nachteil entgegen, daß die Tinte in den Düsenaustrittsöffnungen eines Tintendruckkopfes für längere Schreibpausen der Tintendruckeinrichtung eintrocknet. Um dieses Eintrocknen zu verhindern, weist die Tintendruckeinrichtung beispielsweise eine Reinigungs- und Dichtstation bzw. eine Saug-Regeneriereinrichtung auf. Darüber hinaus ist es die Aufgabe der Reinigungs- und Dichtstation, Verschmutzungen an den Düsenaustrittsöffnungen des Tintendruckkopfes in der Tintendruckeinrichtung zu beseitigen. Hierzu werden die Tintendruckköpfe in regelmäßigen Abständen, wie es schon der Name Reinigungs- und Dichtstation verrät, gereinigt und bei längeren Schreibpausen der Tintendruckeinrichtung abgedichtet. Die an die Reinigungs- und Dichtstation gestellten Anforderungen bedingen Maßnahmen, die das Farbmischen beim Reinigen und Abdichten der Tintendruckköpfe und das Zerstören von Menisken in Düsenaustrittsöffnungen der Tintendruckköpfe beim Ankoppeln einer Saug- und Abdeckeinrichtung an ein Tintendruckwerk verhindern. Die Saug- und Abdeckeinrichtung ist dazu beispielsweise in einem zweiteilig aufgebauten, seitlich verschiebbar ausgebildeten Schwenkhebel angeordnet. Um die Saug- und Abdeckeinrichtung an das Tintendruckwerk ankoppeln und im angekoppelten Zustand Tinte aus den Tintendruckköpfen mit einer Pumpe absaugen zu können, müssen die hierfür notwendigen Bewegungen des Schwenkhebels bzw. der Pumpe erzeugt und voneinander entkoppelbar sein.

Aus den DE-A1-33 16 474, DE-A1-33 16 968, DE-A1-36 04 373, DE-A1-36 11 333, DE-A1-36 33 239, DE-A1-37 26 671, DE-A1-38 10 698 und EP-A1-0 094 220 ist jeweils eine Reinigungs- und Dichtstation bzw. eine Saug-Regeneriereinrichtung für Tintendruckköpfe in Tintendruckeinrichtungen bekannt, mit der auf unterschiedliche Art und Weise Düsenaustrittsöffnungen der Tintendruckköpfe gereinigt, gespült und diese für längere Schreibpausen der Tintendruckeinrichtungen abgedichtet werden. Die Reinigungs- und Dichtstation bzw. Saug-Regeneriereinrichtung ist dazu vorzugsweise in einer Parkposition außerhalb des Arbeitsbereiches eines die Tintendruckköpfe tragenden Druckerwagens in der Tintendruckeinrichtung angeordnet. Der dadurch zusätzlich benötigte Einbauraum in der Tintendruckeinrichtung bewirkt je nach der äußeren Abmessung der Reinigungs- und Dichtstation bzw. Saug-Regeneriereinrichtung, daß die Tintendruckeinrichtung breiter und somit unhandlicher wird.

Aus der DE-A1-36 11 333 ist darüber hinaus bekannt, wie eine Saug- und Abdeckeinrichtung vor- und rückwärts bewegbar in der Reinigungs- und Dichtstation angeordnet ist. Dadurch, daß der Druckerwagen mit den Tintendruckköpfen positionsgenau gegenüber der Reinigungs- und Dichtstation durch die gesonderte Parkposition der Reinigungs- und Dichtstation positioniert werden kann, braucht die Saug- und Abdeckeinrichtung nur vor- und rückwärts bewegbar sein. Hierfür ist in der Reinigungs- und Dichtstation ein Motor vorgesehen, der über ein aufwendiges Getriebe einen Pumpenhebel und die gleitend gelagerte Saug- und Abdeckeinrichtung antreibt und dadurch den Ankoppel- und Absaugvorgang einleitet.

Der vorliegenden Erfindung liegt die Aufgabe zugrunde, eine Vorrichtung zum Erzeugen und Entkoppeln unterschiedlicher Bewegungen bei Reinigungs- und Dichtstationen in Tintendruckeinrichtungen kostengünstig und einfach aufzubauen, mit der eine Saug- und Abdeckkappe der Reinigungs- und Dichtstation zum Reinigen von Tintendruckköpfen an eine Düsenplatte der Tintendruckköpfe heranbewegt bzw. von der Düsenplatte wieder wegbewegt werden kann und mit der im angedockten Zustand der Saug- und Abdeckkappe an die Düsenplatte eine Saugeinrichtung (Faltenbalgpumpe) betätigt werden kann.

Diese Aufgabe wird durch die in dem Patentanspruch 1 angegebenen Merkmale gelöst.

Vorteilhafte Ausführungsformen der Erfindung sind in den Unteransprüchen angegeben.

Ausführungsbeispiele der Erfindung werden anhand der Zeichnungen in den Figuren 1 bis 11 erläutert. Es zeigen:
Figur 1 eine Draufsicht auf eine Tintendruckeinrichtung,
Figur 2 eine perspektivische Darstellung einer Reinigungs- und Dichtstation,
Figur 3 bis 11 verschiedene, von der Drehrichtung abhängige Zustandsdiagramme einer Weichenkupplung zum Erzeugen und Entkoppeln der unterschiedlichen Bewegungen bei der Reinigungs- und Dichtstation.

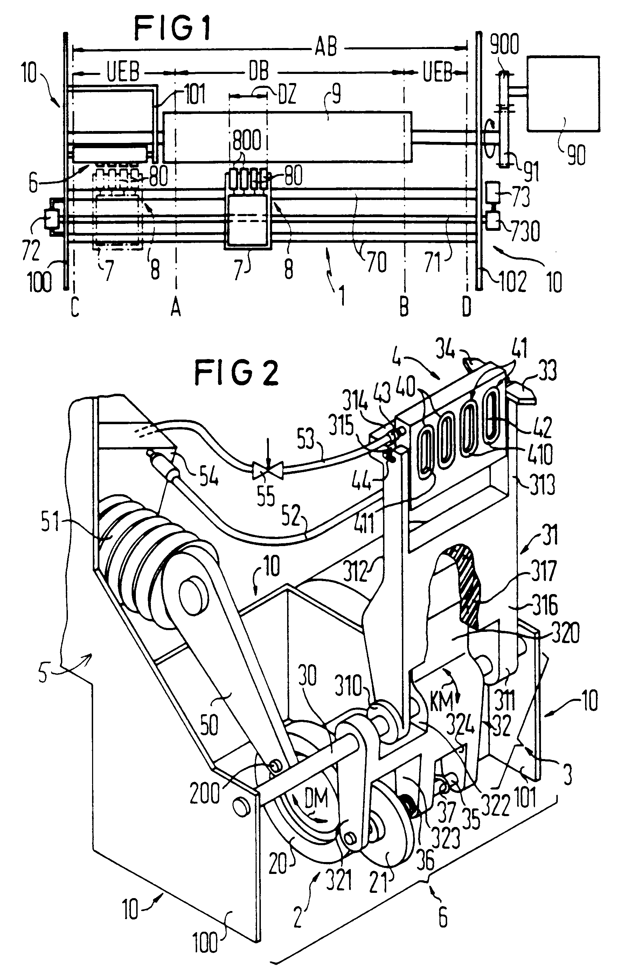

Figur 1 zeigt in einer Draufsicht einen prinzipiellen Aufbau einer Tintendruckeinrichtung 1. Charakteristisch für den Aufbau der Tintendruckeinrichtung 1 ist ein auf einem Druckerwagen 7 angeordnetes Tintendruckwerk 8, das parallel zu einem in zwei Gehäusewänden 100, 102 eines Tragwerkes 10 drehbar gelagerten Druckgegenlager in Form einer Schreibwalze 9 bewegt werden kann. Die Schreibwalze 9, die von einer Antriebseinrichtung 90 mit einem ersten Antriebsritzel 900 über ein Getriebe 91 in der eingezeichneten Drehrichtung angetrieben wird, transportiert einen, sich beispielsweise über einen Druckbereich DB erstreckenden, blattförmigen Aufzeichnungsträger in eine von dem Tintendruckwerk 8 und der Schreibwalze 9 gebildete Druckzone DZ. Um den Aufzeichnungsträger bedrucken zu können, weist das Tintendruckwerk 8 im vorliegenden Fall, wenn die Tintendruckeinrichtung 1 als Vierfarbendrucker ausgebildet ist, vier nebeneinander angeordnete Tintendruckköpfe 80 mit dem Aufzeichnungsträger zugewandten Düsenaustrittsflächen 800 auf. Bei den vier verfügbaren farbigen Schreibflüssigkeiten handelt es sich um die Farben gelb, magenta, cyan und schwarz. Die farbigen Schreibflüssigkeiten können dabei den vier verschiedenen Tintendruckköpfen 80 beliebig zugeordnet werden. Es empfiehlt sich aber aus Gründen, die mit der Reinigung der Tintendruckköpfe 80 zusammenhängen, die Farben in der genannten Reihenfolge den Tintendruckköpfen 80 von rechts nach links zuzuordnen.

Als Druckzone DZ wird der den Tintendruckköpfen 80 gegenüberliegende Bereich des blattförmigen Aufzeichnungsträgers bezeichnet. Um den blattförmigen Aufzeichnungsträger über die gesamte Breite des Druckbereiches DB bedrucken zu können, wird der Druckerwagen 7 auf zwei parallel verlaufenden, in den Gehäusewänden 100, 102 befestigte Führungsstangen 70 hin- und herbewegt. Das Hin- und Herbewegen des Druckerwagens 7 erfolgt dabei, wie in dem DE-GM 89 06 727 beschrieben, durch ein biegsames Zugmittel 71, das eine Umlenkrolle 72 und ein zweites Antriebsritzel 730 eines Elektromotors 73 formschlüssig umschlingt.

Zum Bedrucken des über die Schreibwalze 9 geführten Aufzeichnungsträgers in dem Druckbereich DB wird der Druckerwagen 7 mit dem Tintendruckwerk 8 zwischen den den Druckbereich DB begrenzenden Positionen hin- und herbewegt. Hierbei ist als Betriebsart sowohl ein mono-direktionaler Druckbetrieb als auch ein bidirektionaler Druckbetrieb möglich. Beim mono-direktionalen Druckbetrieb wird der Aufzeichnungsträger nur in einer Bewegungsrichtung zeilenweise beschrieben, beim bidirektionalen Druckbetrieb - der eine wesentlich höhere Druckgeschwindigkeit erlaubt - wird der Aufzeichnungsträger im Druckbereich DB in beiden Bewegungsrichtungen des Tintendruckwerks 8 (Tintendruckkopf) zeilenweise bedruckt.

Unabhängig von der Betriebsart wird bei Druckbeginn der sich in einer Ruheposition C außerhalb des Druckbereiches DB befindliche Druckerwagen 7 zunächst bis zur Position A beschleunigt, damit er die für den kontinuierlichen Druch erforderliche Relativgeschwindigkeit zum Aufzeichnungsträger erreicht. Dabei definiert die Position A die erste mögliche Druckposition. Danach wird im eigentlichen Druckbereich DB der Druckerwagen 7 zum Drucken mit konstanter Geschwindigkeit bewegt bis er die Position B erreicht hat, die die letzte mögliche Druckposition des Druckbereiches DB bestimmt. Nach Überschreiten der Position B wird der Druckerwagen 7 bis zur Position D abgebremst und zum Stillstand gebracht und dann der Aufzeichnungsträger über die Schreibwalze 7 eine Schreibzeile weiterbewegt. Zum Bedrucken der Folgezeile wird der Druckerwagen in umgekehrter Richtung aus der Position D in die Position B beschleunigt, die nunmehr die erste Druckposition der zu bedruckenden Folgezeile festlegt. Nach Erreichen der Druckgeschwindigkeit in der Position B kann die Folgezeile zwischen den Positionen B und A bedruckt werden. Erreicht der Druckerwagen 7 die letzte mögliche Druckposition A wird er bis zur Position C erneut abgebremst. Nunmehr erfolgt ein erneuter Zeilenvorschub mit erneutem Zeilendruck. In der beschriebenen Weise wird nun Zeile für Zeile des Aufzeichnungsträgers bedruckt.

Im mono-direktionalen Betrieb ist es günstig den Druckerwagen im Schnellrücklauf aus der Position B in die Position C zu bewegen.

Die Wegstrecken CA und BD werden in folgendem als Überschwingbereiche UEB bezeichnet, die mit dem Druckbereich DB einen Arbeitsbereich AB für das Tintendruckwerk 8 festlegen. Ihre Mindestlänge ist bestimmt durch die physikalisch erforderlichen Beschleunigungs- und Bremsstrecken unter Berücksichtigung mechanischer Toleranzen. Bei dem im Zusammenhang mit der Fig. 1 beschriebenen Ausführungsbeispiel ist der Überschwingbereich UEB etwa 40 mm lang.

Im Druckbetrieb können durch Papierstaub Verschmutzungen an den Tintendruckköpfen 80 auftreten, deshalb müssen die Tintendruckköpfe 80 von Zeit zu Zeit gereinigt werden. Die Tintendruckköpfe 80 werden dabei gespült, indem Tinte über die Düsenaustrittsöffnungen aus den Tintendruckköpfen 80 abgesaugt wird. Durch das Spülen der Tintendruckköpfe 80 wird auch gleichzeitig verhindert, daß Tinte an Düsenaustrittsöffnungen von Tintendruckköpfen 80, die im Schreibbetrieb nicht benutzt worden sind, eintrocknet. Hierfür ist in der Tintendruckeinrichtung 1 eine Reinigungs- und Dichtstation 6 vorgesehen. Die Reinigungs- und Dichtstation 6 ist in einem Überschwingbereich UEB des Druckerwagens 7 angeordnet. Dies kann sowohl ein linksseitiger als auch ein rechtsseitiger Überschwingbereich UEB sein. Als vorteilhaft hat sich der linksseitige Überschwingbereich herausgestellt.

Für das Reinigen der Tintendruckköpfe 80 wird der Druckerwagen 7 bis zur Anlage an die Gehäusewand 100 des Tragwerkes 10 in den Überschwingbereich UEB gefahren. Die Gehäusewand 100 bildet dabei für die Reinigungs- und Dichtstation 6 und den Druckerwagen 7 eine gemeinsame Bezugskante, die für den Reinigungsvorgang von Bedeutung ist. Wie der Reinigungsvorgang im einzelnen abläuft, wird anhand der Beschreibung der Figuren 2 bis 11 erläutert.

Figur 2 zeigt in perspektivischer Darstellung den Aufbau der Reinigungs- und Dichtstation 6, im folgenden als RD-Station bezeichnet. Die RD-Station 6 ist dabei als autonome, unabhängig von der Tintendruckeinrichtung 1 funktionierende Baueinheit konzipiert. Sie kann als geschlossene Baueinheit in die Tintendruckeinrichtung eingesetzt werden. Dies hat den Vorteil, daß die RD-Station 6 als OEM-Produkt (Original Equipment Manufacturing) in verschiedenen Tintendruckeinrichtungen verwendet werden kann. Mit ihr werden Service-Behandlungen der Tintendruckköpfe 80 vorgenommen, die für einen störungsfreien Betrieb der Tintendruckeinrichtung 1 notwendig sind. Diese umfassen u.a.: Das Reinigen des Tintendruckkopfes 80 mit seinen Düsenaustrittsöffnungen in vorgegebenen Zeitintervallen, um dadurch ein Eintrocknen und Verschmutzen der Düsenaustrittsöffnungen zu verhindern; das Absaugen von im Tintendruckkopf 80 enthaltener Tinte bei Störungen, um z.B. eingedrungene Luft zu entfernen und - im Ruhezustand der Tintendruckeinrichtung 1 - das Abdecken der Düsenaustrittsöffnungen, um diese vor dem Eintrocknen und Verschmutzen, z.B. durch Verstauben durch Papierstaub zu schützen. Darüber hinaus muß beim Transportieren und Lagern der Tintendruckeinrichtung 1 vermieden werden, daß Tinte aus den Düsenaustrittsöffnungen ausläuft.

Da die RD-Station 6 nach Figur 1 innerhalb des aus dem Schreibbetrieb der Tintendruckeinrichtung 1 resultierenden Überschwingbereiches UEB für den das Tintendruckwerk 8 tragenden Druckerwagen 7 in der Tintendruckeinrichtung 1 angeordnet ist, ergibt sich ein schmalerer Aufbau der Tintendruckeinrichtung 1.

Im Schreibbetrieb, wenn sich der Druckerwagen 7 durch Beschleunigungs- und Bremsvorgänge zeitweilig in dem Überschwingbereich UEB aufhält, darf die RD-Station 6 die Überschwingstrecke des Druckerwagens 7 zwischen der Position A und der Position C nach Figur 1 nicht versperren.

Im Servicebetrieb, wenn die Düsenaustrittsöffnungen des Tintendruckwerkes 8 gereinigt werden sollen, muß die RD-Station 6 positionsgenau an das Tintendruckwerk 8 angedockt und die Tinte aus den Düsenaustrittsöffnungen abgesaugt werden. Unter "Andocken" wird dabei ein Ankoppeln der RD-Station 6 an das Tintendruckwerk 8 verstanden.

Im Ruhezustand, beim Transport und bei der Lagerung der Tintendruckeinrichtung 1 müssen die Düsenaustrittsöffnungen vor dem Eintrocknen geschützt werden. Weiterhin darf keine Tinte auslaufen. Deswegen ist es notwendig, die RD-Station 6 positionsgenau an das Tintendruckwerk 8 anzudocken und damit die Düsenaustrittsöffnungen zu verschließen.

Die RD-Station 6 enthält eine Weichenkupplung 2, einen Schwenkhebel 3, eine Saug- und Abdeckkappe 4, im folgenden als SA-Kappe bezeichnet, sowie eine Faltenbalgpumpe 5. Die Weichenkupplung 2 weist eine Nockenscheibe 20 und ein Laufrad 21 auf, das sich auf der Nockenscheibe 20 abwälzt. Die Nockenscheibe 20 ist zur Aufnahme eines Drehmomentes DM auf einer Antriebswelle eines in der Figur 2 nicht dargestellten, weiteren Elektromotors formschlüssig befestigt. Als Elektromotor wird dabei vorzugsweise ein Gleichstrommotor verwendet.

Weiterhin weist die Nockenscheibe 20 auf der dem Elektromotor abgewandten Stirnflächenseite einen exzentrisch angeordneten, vorspringenden Kurbelzapfen 200 auf, der über ein Gestänge 50 mit einem Faltenbalg 51 der Faltenbalgpumpe 5 verbunden ist. Durch die Drehung der Nockenscheibe 20 mit dem exzentrisch angeordneten Kurbelzapfen 200 wird der Faltenbalg 51 über das Gestänge 50 abwechselnd auseinander gezogen bzw. zusammengedrückt. Die dadurch entstehende Pumpwirkung der Faltenbalgpumpe 5 wird bei der vorliegenden RD-Station 6 dazu benutzt, um beispielsweise die Tinte aus den Düsenaustrittsöffnungen der Tintendruckköpfe 80 in Figur 1 abzupumpen. Die Faltenbalgpumpe 5 ist dazu sowohl über einen Schlauch 52 als auch über einen Luftschlauch 53 mit der SA-Kappe 4 verbunden. Mit der RD-Station 6 ist aber auch möglich, andere Flüssigkeiten aus diversen Spritzpinrichtungen abzusaugen und zu entsorgen.

Für das Abpumpen der Tinte aus den Tintendruckköpfen 80 der Tintendruckeinrichtung 1 sind in der SA-Kappe 4 vier gleich große, identisch ausgeformte Ausnehmungen (Absaugöffnungen, Hohlräume) 40 angeordnet. Diese sind einerseits über den Schlauch 52 (Absaugkanal) mit einem an der Faltenbalgpumpe 5 befestigten Entsorgungsbehälter 54 verbunden, andererseits über den Luftschlauch 53 (Druckausgleichskanal) mit der Umgebungsluft. Der Luftschlauch weist ein z.B. mit der Faltenbalgpumpe 5 gekoppeltes, steuerbares Belüftungsventil 55 auf. Der Luftschlauch 53 ist dazu über einen Belüftungsstutzen 43 gestülpt, der seitlich aus der SA-Kappe 4 herausragt. Die Tinte kann alternativ zur Faltenbalgpumpe 5 auch mit einer Schlauch-, Kolben- und Membranpumpe aus den Düsenaustrittsöffnungen abgesaugt werden.

Die Anzahl der Ausnehmungen 40, die in der SA-Kappe 4 der RD-Station 6 enthalten sind, richtet sich nach der Zahl der verwendeten Tintendruckköpfe. Wenn, wie im vorliegenden Fall, mit der Tintendruckeinrichtung 1 beispielsweise ein Vierfarben-Druckbild hergestellt werden soll, muß auch die Service-Behandlung der Tintendruckeinrichtung 1 für die Entsorgung der erforderlichen vier Tintendruckköpfe ausgelegt sein. Um ein Mischen der Schreibflüssigkeiten während des Absaugens aus den Düsenaustrittsöffnungen der Tintendruckköpfe zu vermeiden, ist die Anzahl der Ausnehmungen 40 bzw. Absaugöffnungen identisch mit der Anzahl der verwendeten, den Tintendruckköpfen zugeordneten farbigen Schreibflüssigkeiten.

Auf der dem Tintendruckwerk 8 zugewandten Seite der SA-Kappe 4 ist in den Absaugöffnungen 40 jeweils ein wannenförmiger Gummieinsatz 41 in Form einer elastischen Kappe angeordnet. Dieser weist eine flüssigkeitsabsorbierende Einlage 42 auf. Für das Abpumpen der Tinte aus den Tintendruckköpfen 80 wird die SA-Kappe 4, wie bereits erwähnt, an das Tintendruckwerk 8 angedockt, wobei die elastischen Kappen 41 sich über die Düsenaustrittsöffnungen legen. Wenn im folgenden vom Andocken der SA-Kappe 4 die Rede ist, so ist damit ein seitliches Verschieben und Schwenken der SA-Kappe 4 gemeint. Damit die Tinte über die Absaugöffnungen 40 sowie den Schlauch 52 einwandfrei abgepumpt werden kann, ist auf dem wannenförmigen Gummieinsatz 41 eine Dichtungslippe 410 angeordnet, die eine Wannenöffnung 411 des Gummieinsatzes 41 umgibt und beim Andocken der SA-Kappe 4 gegen das Tintendruckwerk 8 gedrückt wird und dabei die Düsenaustrittsöffnungen der Tintendruckköpfe 80 hermetisch abdichtet.

Das Andocken der SA-Kappe 4 wird durch den Schwenkhebel 3 bewerkstelligt, der auf einer zwischen der Gehäusewand 100 und einer weiteren Gehäusewand 101 des Tragwerkes 10 eingespannten ersten Achse 30 verschieb- und schwenkbar gelagert ist. Um das Verschieben und Schwenken des Schwenkhebels 3 mit minimalen Kraftaufwand vornehmen zu können, müssen Reibungseinflüsse so klein wie möglich gehalten werden.

Der Schwenkvorgang wird dadurch ausgelöst, daß das von dem Elektromotor abgegebene Drehmoment DM über die Weichenkupplung 20 in ein an dem Schwenkhebel 3 angreifendes Kippmoment KM umgewandelt wird. Für die Umwandlung des Drehmomentes DM ist der Schwenkhebel 3 über das Laufrad 21 an die Nockenscheibe 20 angefedert. Um die bei der Anfederung auftretenden Kräfte klein zu halten, ist der Schwenkhebel 3 zweiteilig aufgebaut und damit wird auch das für die Reibungseinflüsse mitverwantwortliche Eigengewicht des Schwenkhebels 3 aufgeteilt. Der zweiteilige Aufbau des Schwenkhebels 3 erklärt sich jedoch im wesentlichen dadurch, daß für ein positionsgenaues Andocken der SA-Kappe 4 eine seitliche Verschiebung des Schwenkhebels 3 erforderlich sein kann. Bei einem einteiligen Aufbau würde dies zu einem Verschieben des Laufrades 21 auf der Nockenscheibe 20 führen.

Die Laufoberfläche für das Laufrad 21 auf der Nockenscheibe 20 müßte bei einem einteiligen Aufbau für eine maximal auftretende seitliche Verschiebung beim Andocken ausgelegt sein.

Ein die SA-Kappe 4 tragendes Hebeloberteil 31 des Schwenkhebels 3 ist über zwei Schwenkarme 310, 311 schwenk- und verschiebbar auf der Achse 30 angeordnet. Das Hebeloberteil 31 des Schwenkhebels 3 weist weiterhin zwei sich gegenüberliegende Stützarme 312, 313 auf, die auf der dem Tintendruckwerk 8 abgewandten Seite über eine u-förmige Querverstrebung 314 miteinander verbunden sind. In den Schenkeln der u-förmigen Querverstrebung 314 ist in einer ersten Ausführungsform für die Lagerung der SA-Kappe 4 jeweils eine T-förmige Ausnehmung 315 eingelassen. Diese dient zur freibeweglichen Lagerung von Lagerzapfen 44 der SA-Kappe 4. Zur Lagerung der SA-Kappe 4 werden die Lagerzapfen 44 in die T-förmige Ausnehmung 315 gedrückt. Zwischen den Schwenkarmen 310, 311 und den Stützarmen 312, 313 weist das Hebeloberteil 31 noch ein rechteckförmiges Mittelteil 316 auf, in das eine taschenförmige Ausformung 317 eingelassen ist.

Damit die SA-Kappe 4 auch positionsgenau an das Tintendruckwerk 8 angedockt werden kann, ist eine Postioniereinrichtung vorgesehen. Diese besteht aus zwei sich gegenüberliegenden, in Schwenkrichtung des Schwenkhebels 3 spitzwinklig zulaufenden Zentrierfingern 33, die auf der Seite des Stützarms 313 auf dem Schenkel der U-förmigen Querverstrebung 314 angeordnet sind. Ein erster Zentrierfinger 33 sucht sich dazu beim Schwenken des Schwenkhebels 3 selbständig ein in das Tintendruckwerk 8 eingelassenes erstes, in der Figur 2 nicht dargestelltes Zentrierfenster und positioniert so die RD-Station 6 gegenüber dem Tintendruckwerk 8. Zum Positionieren kann der Schwenkhebel 3 mit der RD-Station 6 seitlich verschoben werden.

Für das Andocken der SA-Kappe 4 wird das Kippmoment KM über ein Hebelunterteil 32 des Schwenkhebels 3 auf das Hebeloberteil 31 des Schwenkhebels 3 übertragen. Das Hebelunterteil 32 ist dazu, wie das Hebeloberteil 31, auf der Achse 30 schwenkbar angeordnet. Charakteristisch für das Hebelunterteil 32 sind ein Hebelarm 320 und ein Nebenarm 321, durch die die Achse 30 mittig bzw. am Fußpunkt durchgesteckt ist. Zwischen dem Hebelarm 320 und dem Nebenarm 321 ist im Bereich der Achse 30 eine erste Aussparung 322 vorgesehen, in der der Schwenkarm 310 des Hebeloberteils 31 angeordnet ist. Die Abmaße der Aussparung 322 sind dabei so gewählt, daß das Hebeloberteil 31 unabhängig vom Hebelunterteil 32 je nach Bedarf seitlich verschoben werden kann. Darüber hinaus ist zwischen dem Hebelarm 320 und dem Nebenarm 321 eine zweite Aussparung 323 vorgesehen, in der das Laufrad 21 auf einer zweiten Achse 35 axial bewegbar und drehbar gelagert ist. Auf der Achse 35 innerhalb der Aussparung 323 ist weiterhin noch eine erste Feder 36 angeordnet, die der axialen Beweglichkeit des Laufrades 21 mit einer ersten Federkraft F1 entgegenwirkt. Die Achse 35 durchgreift außerdem noch eine dritte Aussparung 324, die am Fußpunkt des Hebelarms 320 eingelassen ist. Innerhalb dieser dritten Aussparung 324 ist an der Achse 35 eine zweite Feder 37 mit einer Federkraft F2 eingehängt, die für den Schwenkvorgang des Schwenkhebels 3 außerdem noch, jedoch in der Figur 1 nicht sichtbar dargestellt, mit dem Tragwerk 10 der Tintendruckeinrichtung 1 verbunden ist. Durch die zweite Federkraft F2 der Feder 37 wird das Laufrad 21 gegen die Nockenscheibe 20 gedrückt. Die für das Andocken der SA-Kappe 4 erforderliche Schwenkbewegung des Schwenkhebels 3 wird von dem Hebelarm 320 des Hebelunterteils 32 auf das Hebeloberteil 31 übertragen. Der Hebelarm 320 greift dazu mit einem kleinen Spiel in Schwenkrichtung des Schwenkhebels 3 gleitsteinartig in die taschenförmige Ausformung 317 des Hebeloberteils 31 zwischen den Schwenkarmen 310, 311 ein. Um das Hebeloberteil 31 auf der Achse 30 verschieben zu können, ist die taschenförmige Ausformung 317 des Hebeloberteils 31 gegenüber dem Hebelarm 320 des Hebelunterteils 32 um jenen Betrag breiter, welcher als seitliche Verschiebung des Hebeloberteils 31 und somit der SA-Kappe 4 benötigt wird.

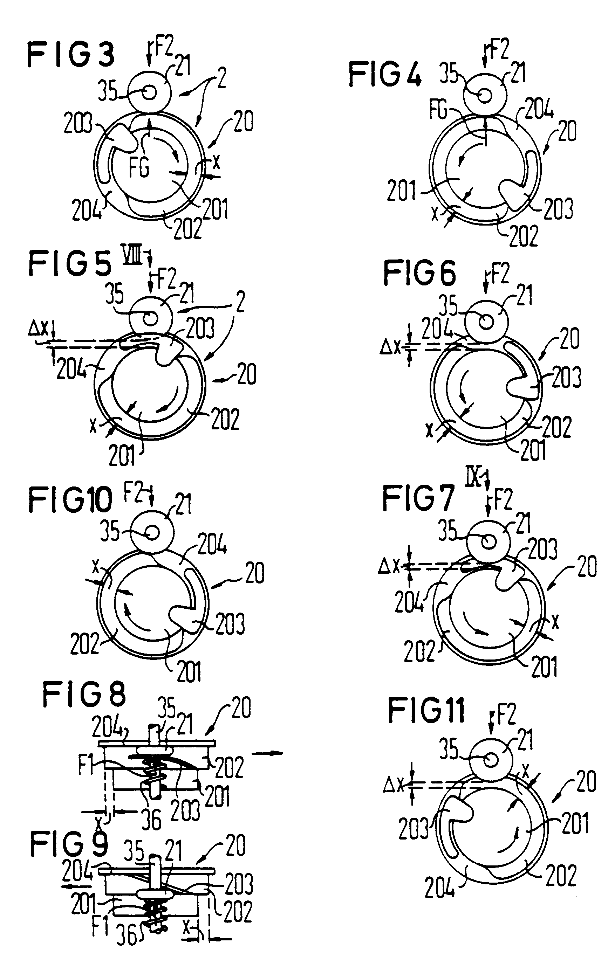

Figur 3 bis 7 sowie Figur 10 und 11 zeigen in einer Seitenansicht den Aufbau und die Wirkungsweise der Weichenkupplung 2 anhand von drehwinkelabhängigen Zuständen der Weichenkupplung 2 für unterschiedliche Drehrichtungen der Nockenscheibe 20. Die beiden Drehrichtungen der Nockenscheibe 20 werden ausgenutzt, um die bei der Beschreibung der Figur 2 genannten Betriebsarten der RD-Station 6 zu realisieren. Bei der Rechtsdrehung der Nockenscheibe 20 gemäß den Figuren 3, 5 und 10 wird die im Schwenkhebel 3 integrierte SA-Kappe 4 im ständigen Wechsel an das Tintendruckwerk 8 angedockt (Figur 3) und wieder vom Tintendruckwerk 8 abgehoben (Figur 10). Bei der Linksdrehung der Nockenscheibe 20 gemäß den Figuren 4, 6, 7 und 11 wird der Schwenkhebel 3 an das Tintendruckwerk angedockt (Figur 4) und die Tinte aus den Düsenaustrittsöffnungen des Tintendruckkopfes durch die Faltenbalgpumpe 5 solange abgesaugt (Figuren 6, 7, 11) bis die Drehrichtung wieder gewechselt wird.

Für den Schwenk- bzw. Absaugvorgang weist die Nockenscheibe 20 zwei radial verlaufende, um einen Stufenabsatz x voneinander abgesetzte Nockenbahnen 201, 202 auf, bei der eine äußere, bewegungsauslösende Nockenbahn 202 nach Figuren 3, 4 und 10 als Lauffläche für das sich auf der Nockenscheibe 20 abwälzende Laufrad 21 dient. Um einen für das Andocken der SA-Kappe 4 an das Tintendruckwerk 8 und einen für das Abheben der SA-Kappe 4 von dem Tintendruckwerk 8 erforderlichen Hub ausführen zu können, ist der volle Stufenabsatz x zwischen den Nockenbahnen 201, 202 beispielsweise für zwei Drittel des Umfangs auf der Nockenscheibe 20 wirksam. Dadurch, daß sich die bewegungsauslösende, äußere Nockenbahn 202 an diesen Stellen an eine innere als Leerlaufbahn ausgebildete Nockenbahn 201 anschmiegt, wird die Position des Laufrads 21 unter der Federkraft F2 bzw. gegen die Federkraft F2 um den Betrag für den Hub gegenüber der Nockenscheibe 20 verschoben.

In Figur 3 und 4 ist jeweils ein möglicher Ausgangszustand der Weichenkupplung 2 dargestellt, bei dem das Laufrad 21 auf der äußeren Nockenbahn 202 der Nockenscheibe 20 aufliegt und sich somit die im Schwenkhebel 3 integrierte SA-Kappe 4 nach Figur 2 im abgehobenen Zustand vom Tintendruckwerk 8 befindet. Eine die Federkraft F2 kompensierende Gleichgewichtskraft FG wird dabei von der drehbar gelagerten Nockenscheibe 20 aufgenommen. Das Laufrad 21, der Schwenkhebel 3 und die SA-Kappe 4 bilden eine doppelarmige mechanische Hebelanordnung, bei der die auf die Hebelanordnung wirkende Federkraft F2 entweder über das Laufrad 20 oder die SA-Kappe 4 kompensiert wird. Wird nun die Nockenscheibe 20 in der eingezeichneten Pfeilrichtung gemäß Figur 3 nach rechts oder gemäß Figur 4 nach links gedreht, so verläßt das Laufrad 21 unter der Federkraft F2 in beiden Fällen die äußere Nockenbahn 202 der Nockenscheibe 20. Die dadurch entstehende Positionsverschiebung des Laufrades 21 bewirkt nach Figur 2 das Andocken der SA-Kappe 4 an das Tintendruckwerk 8, indem der Schwenkhebel 3 um den Hub geschwenkt wird.

Je nach Zustand der Tintendruckeinrichtung 1, z. B. Schreib- oder Servicebetrieb sowie Ruhezustand, muß für die vorgegebene Drehrichtung der Nockenscheibe 20 nun entweder der, beispielsweise bei der Linksdrehung der Nockenscheibe 20, Pumpvorgang oder das Abheben der SA-Kappe 4 veranlaßt werden. Hierfür ist eine nach dem Kupplungsprinzip arbeitende Weichenzunge 203 vorgesehen. Diese ist im Bereich der Nockenscheibe 20 außerhalb des Stufenabsatzes x angeordnet. Sie überstreicht die innere Nockenbahn 201 schräg, ist an einem Ende mit der Nockenscheibe 20 fest verbunden und liegt am anderen Ende federnd an einem Bord 204 der Nockenscheibe 20 an. Die Weichenzunge 203 ist so dimensioniert und auf der Nockenscheibe 20 so angeordnet, daß das Laufrad 21 bei der Linksdrehung der Nockenscheibe 20 keine weitere Positionsverschiebung vollzieht, das Laufrad 21 also relativ zur bewegten Nockenscheibe 20 leerläuft, und bei der Rechtsdrehung der Nockenscheibe 20 pro Umdrehung zwei Positionsverschiebungen ausführen kann.

Die richtige Auswahl der Drehrichtung in Abhängigkeit von der Betriebsart der Tintendruckeinrichtung 1 wird dabei von einer Steuereinrichtung vorgenommen, die, wie die RD-Station 6, Bestandteil der Tintendruckeinrichtung 1 ist. Die Steuereinrichtung ist dazu mit dem Elektromotor gekoppelt. Für die Steuerung des Elektromotors enthält die Steuereinrichtung beispielsweise einen Mikroprozessor, der über eine allgemein bekannte elektronische Schaltungsanordnung die Polarität einer an den Elektromotor angelegten Versorgungsspannung und dadurch die Drehrichtung des Elektromotors ändert. Neben der Steuerung des Elektromotors der RD-Station 6 werden auch der Elektromotor 73 und die Antriebseinrichtung 90 nach Figur 1 von der Steuereinrichtung gesteuert. Die Steuereinrichtung ist im allgemeinen in üblicher Weise aufgebaut.

Figur 5, 6 und 7 zeigt jeweils einen Zustand der Weichenkupplung 2, bei dem das Laufrad 21 die äußere Nockenbahn 202 verlassen hat und somit die SA-Kappe 4 an das Tintendruckwerk 8 angedockt ist. Charakteristisch für diesen Zustand in Figur 5, 6 und 7 ist es, daß das Laufrad 21 im angedockten Zustand der SA-Kappe 4 weder auf der äußeren Nockenbahn 202 noch auf der inneren Nockenbahn 201 der Nockenscheibe 20 aufliegt. Wenn das Laufrad 21 die äußere Nockenbahn 202 verläßt, ist die mechanische Hebelanordnung unter dem Einfluß der Federkraft F2 bestrebt, wieder einen Auflagezustand einzunehmen. Dies wird dadurch erreicht, daß im Unterschied zur Figur 3 und 4, wo die die Federkraft F2 kompensierende Gleichgewichtskraft FG von der drehbar gelagerten Nockenscheibe 20 aufgebracht wurde, die die Federkraft F2 kompensierende Gleichgewichtskraft FG nun von dem Tintendruckwerk 8 aufgebracht wird. Das bedeutet, daß der von dem Schwenkhebel 3 für das Andocken der SA-Kappe 4 an das Tintendruckwerk 8 auszuführende Hub kleiner ist als der Stufenabsatz x zwischen den Nockenbahnen 201, 202 der Nockenscheibe 20. Es ergibt sich somit ein Luftspalt x, um den das Laufrad 21 von der inneren Nockenbahn 201 der Nockenscheibe 20 abgehoben ist. Durch den Luftspalt x wird erreicht, daß beim Bahnwechsel des Laufrades 21 gemäß Figur 6 und 7 kein zusätzlicher Reibungseinfluß auftritt. Demzufolge kann die Federkraft F1, die das Laufrad 21 gegen den Bord 204 der Nockenscheibe 20 drückt, klein sein. Ein typischer Hubwert für die RD-Station 6 liegt beispielsweise zwischen 6 und 10 mm. Während das Laufrad 21 nach Figur 5 relativ zur Nockenscheibe 20 zwischen dem Bord 204 und der Weichenzunge 203 bewegt wird, wird das Laufrad 21 nach Figur 6 und 7 relativ zur Nockenscheibe 20 an der Weichenzunge 203 vorbeigeführt.

Figur 8 und 9 zeigt jeweils in einer Draufsicht den Zustand der Weichenkupplung 2 nach Figur 5 bzw. Figur 7. Dabei wird verdeutlicht, daß das auf der Achse 35 durch die Feder 36 mit der Federkraft F1 gegen den Bord 204 der Nockenscheibe 20 gedrückte Laufrad 21 bei der Rechtsdrehung der Nockenscheibe 20 in Figur 8 zwischen der Weichenzunge 203 und dem Bord 204 relativ zur Nockenscheibe 20 hindurch bewegt und bei der Linksdrehung der Nockenscheibe 20 in Figur 9 an der Weichenzunge 203 relativ zur Nockenscheibe 20 vorbeigeführt wird.

Bei der Relativbewegung des Laufrades 21 nach Figur 8 wird das sich federnd gegen den Bord 204 stemmende Ende der Weichenzunge 203 von dem Laufrad 21 weggedrückt.

Bei der Relativbewegung des Laufrades 21 nach Figur 9 wird das Laufrad 21 auf der Achse 35 verschoben und dabei die Feder 36 zusammengedrückt. Die Feder 36 wird dabei solange von dem Laufrad 21 zusammengedrückt, bis das Laufrad 21 die Weichenzunge 203 passiert hat.

In Figur 10 ist dargestellt, wie das Laufrad 21 bei weiterer Rechtsdrehung der Nockenscheibe 20 gegen die Federkraft F2 wieder auf die äußere Nockenbahn 202 gelangt und dadurch die SA-Kappe 4 von dem Tintendruckwerk 8 abgehoben wird. Bei weiterer Rechtsdrehung der Nockenscheibe 20 wird wieder der in Figur 3 dargestellte Zustand der Weichenkupplung 2 erreicht und das Andocken bzw. Abheben der SA-Kappe 4 von neuem begonnen.

Figur 11 zeigt, wie das Laufrad 21 bei weiterer Linksdrehung der Nockenscheibe 20 nach dem Passieren der Weichenzunge 203 in Figur 7 und 9 durch die Federkraft F1 gegen den Stufenabsatz x der voneinander ab gesetzten Nockenbahnen 201, 202 gedrückt wird. Bei weiterer Linksdrehung der Nockenscheibe 20 wird wieder der in Figur 6 dargestellte Zustand der Weichenkupplung 2 erreicht und die Tinte aus den Düsenaustrittsöffnungen der Tintendruckköpfe 80 weiter abgepumpt.

### Bezugszeichenliste

- 1: Tintendruckeinrichtung
- 2: Weichenkupplung
- 3: Schwenkhebel
- 4: Saug- und Abdeckkappe (SA-Kappe)
- 5: Faltenbalgpumpe
- 6: Reinigungs- und Dichtstation (RD-Station)
- 7: Druckerwagen
- 8: Tintendruckwerk
- 9: Schreibwalze
- 10: Tragwerk
- 20: Nockenscheibe
- 21: Laufrad
- 30: erste Achse
- 31: Hebeloberteil
- 32: Hebelunterteil
- 33: erster Zentrierfinger
- 34: zweiter Zentrierfinger
- 35: zweite Achse
- 36: erste Feder
- 37: zweite Feder
- 40: Hohlraum, Ausnehmnung, Absaugöffnung
- 41: wannenförmiger Gummieinsatz
- 42: flüssigkeitsabsorbierende Einlage
- 43: Belüftungsstutzen
- 44: Lagerzapfen
- 50: Gestänge
- 51: Faltenbalg
- 52: Schlauch
- 53: Luftschlauch
- 54: Entsorgungsbehälter
- 55: Belüftungsventil
- 70: Führungsstange
- 71: biegsames Zugmittel
- 72: Umlenkrolle
- 73: Elektromotor
- 80: Tintendruckkopf
- 81: erstes Zentrierfenster
- 90: Antriebseinrichtung
- 91: Getriebe
- 100, 101, 102: Gehäusewand
- 200: Kurbelzapfen
- 201: innere Nockenbahn
- 202: äußere Nockenbahn
- 203: Weichenzunge
- 204: Bord
- 310, 311: Schwenkarm des Hebeloberteils
- 312, 313: Stützarm des Hebeloberteils
- 314: Querverstrebung
- 315: -förmige Ausnehmung
- 316: Mittelteil des Hebeloberteils
- 317: taschenförmige Ausformung
- 320: Hebelarm des Hebelunterteils
- 321: Nebenarm des Hebelunterteils
- 322: erste Aussparung
- 323: zweite Aussparung
- 324: dritte Aussparung
- 410: Dichtungslippe
- 411: Wannenöffnung
- 730: zweites Antriebsritzel
- 800: Düsenaustrittsfläche
- 900: erstes Antriebsritzel
- A, B, C, D: Zustände des Druckerwagens
- AB: Arbeitsbereich des Druckerwagens
- DB: Druckbereich
- DM: Drehmoment
- DZ: Druckzone
- F1, F2: Federkraft
- KM: Kippmoment
- UEB: Überschwingbereich des Druckerwagens
- a: Zentrierweg
- x: Stufenabstand
- x: Luftspalt

## Patentansprüche

1. Vorrichtung zum Erzeugen und Entkoppeln unterschiedlicher Bewegungen bei Reinigungs- und Dichtstationen in Tintendruckeinrichtungen mit folgenden Merkmalen:
a) eine motorisch angetriebene Nockenscheibe (20) weist einen axial vorspringenden, exzentrisch angeordneten Kurbelzapfen (200) auf, mit dem eine erste Bewegung erzeugt wird,
b) die motorisch angetriebene Nockenscheibe (20) weist zwei stufig voneinander abgesetzte Nockenbahnen (201, 202) auf, die an zwei Stellen der Nockenscheibe (20) ineinander übergehen,
c) ein durch die Nockenbahnen (201, 202) geführtes Laufrad (21) ist zum Erzeugen einer zweiten Bewegung in radialer Richtung zur Nockenscheibe (20) durch eine Federkraft (F2) federnd auf der Nockenscheibe (20) angeordnet,
d) auf der Nockenscheibe (20) ist eine Weichenzunge (203) angeordnet, die durch Steuern der Umlaufbahn des Laufrades (21) um die Nockenscheibe (20) in Abhängigkeit von der Drehrichtung der motorisch angetriebenen Nockenscheibe (20) die zweite Bewegung von der ersten Bewegung entkoppelt.

2. Vorrichtung nach Anspruch 1, **dadurch gekennzeichnet,** daß die Weichenzunge (203) als eine selbststeuernde Schaltzunge ausgebildet ist.

3. Vorrichtung nach Anspruch 1 oder 2, **dadurch gekennzeichnet,** daß die Weichenzunge (203) zwischen den beiden Stellen auf der Nockenscheibe (20) angeordnet ist, wo eine erste Nockenbahn (201) in eine zweite Nockenbahn (202) übergeht.

4. Vorrichtung nach einem der Ansprüche 1 bis 3, **dadurch gekennzeichnet,** daß die Weichenzunge (203) mit einem Ende an einer von den beiden Stellen, wo die erste Nockenbahn (201) in die zweite Nockenbahn (202) übergeht, an der Nockenscheibe (20) befestigt ist und mit dem gegenüberliegenden Ende die beiden Nockenbahnen (201, 202) überstreichend federnd gegen einen Bord (204) der Nockenscheibe (20) stößt.

5. Vorrichtung nach Anspruch 1, **dadurch gekennzeichnet,** daß das Laufrad (21) gegen eine axiale Federkraft (F1) von den Nockenbahnen (201, 202) geführt wird.

6. Vorrichtung nach Anspruch 1 oder 5, **dadurch gekennzeichnet,** daß das Laufrad (21) für das Erzeugen der zweiten Bewegung unter der Einwirkung der Weichenzunge (203) abwechselnd auf der ersten Nockenbahn (202) aufliegt und um einen Luftspalt ( x) von der zweiten Nockenbahn (201) abgehoben ist.

7. Vorrichtung nach Anspruch 1 oder 5, **dadurch gekennzeichnet,** daß das Laufrad (21), wenn die zweite Bewegung von der ersten Bewegung entkoppelt wird, um den Luftspalt ( x) von der zweiten Nockenbahn (201) abgehoben ist.

## Claims

1. A device for the production and decoupling of different movements in cleaning- and sealing stations in ink printing devices with the following features:
a) a motor-driven cam disc (20) has an axially projecting, eccentrically arranged crankpin (200), with which a first movement is produced,
b) the motor-driven cam disc (20) has two cams (201, 202) recessed from each other in a graduated manner, which continue into each other at two sites of the cam disc (20),
c) a runner wheel (21) guided through the cams (201, 202) is arranged elastically on the cam disc (20) to produce a second movement in radial direction to the cam disc (20) by an elastic force (F2),
d) on the cam disc (20) a switch blade (203) is arranged, which through controlling the circulatory path of the runner wheel (21) about the cam disc(20) as a function of the direction of rotation of the motor-driven cam disc (20) decouples the second movement from the first movement.

2. A device according to Claim 1, characterised in that the switch blade (203) is constructed as a self-controlling switch blade.

3. A device according to Claim 1 or 2, characterised in that the switch blade (203) is arranged between the two sites on the cam disc (20), where a first cam (201) continues into a second cam (202).

4. A device according to one of Claims 1 to 3, characterised in that the switch blade (203) is attached to the cam disc (20) by one end at one of the two sites where the first cam (201) continues into the second cam (202) and at the opposite end, sweeping over the two cams (201,202), strikes elastically against an edge (204) of the cam disc (20).

5. A device according to Claim 1, characterised in that the runner wheel (21) is guided against an axial elastic force (F1) by the cams (201, 202).

6. A device according to Claim 1 or 5, characterised in that the runner wheel (21) for the production of the second movement under the action of the switch blade (203) lies alternately on the first cam (202) and is raised by an air gap (x) from the second cam (201).

7. A device according to Claim 1 or 5, characterised in that the runner wheel (21), when the second movement is decoupled from the first movement, is raised by the air gap (x) from the second cam (201).

## Revendications

1. Dispositif pour engendrer et découpler différents mouvements dans les postes de nettoyage et d'étanchéification dans des dispositifs d'impression à encre, comportant les caractéristiques suivantes :
a) un disque à cames (20) entraîné par moteur comporte un tourillon de vilebrequin saillant (200) agencé de façon excentrique, par lequel est engendré un premier mouvement,
b) le disque à cames (20) entraîné par moteur comporte deux voies à cames (201, 202) décalées sous forme d'étage l'une de l'autre et passant l'une dans l'autre à deux endroits du disque à cames (20),
c) une roue de roulement (21) guidée dans les voies à cames (201, 202) est agencée, pour engendrer un second mouvement, en direction radiale par rapport au disque à cames (20), de façon élastique selon une force élastique (F2), sur le disque à cames (20),
d) sur le disque à cames (20) est agencée une languette (203) qui, par la commande de la voie de rotation de la roue de roulement (21) autour du disque à cames (20), en dépendance du sens de rotation du disque à cames (20) entraîné par moteur, découple le second mouvement du premier mouvement.

2. Dispositif selon la revendication 1,
caractérisé en ce que la languette (203) est réalisée comme languette de commutation à commande automatique.

3. Dispositif selon l'une des revendications 1 ou 2,
caractérisé en ce que la languette (203) est agencée entre les deux endroits sur le disque à cames (20) où une première voie à cames (201) passe dans une seconde voie à cames (202).

4. Dispositif selon l'une des revendications 1 à 3,
caractérisé en ce que la languette (203) est fixée par une extrémité, à l'un des deux endroits où la première voie à cames (201) passe dans la seconde voie à cames (202), sur le disque à cames (20), et pousse par l'extrémité opposée les deux voies à cames (201, 202) de façon superficielle et élastique contre un bord (204) du disque à cames (20).

5. Dispositif selon la revendication 1,
caractérisé en ce que la roue de roulement (21) est guidée par les voies à cames (201, 202) contre une force axiale élastique (F1).

6. Dispositif selon l'une des revendications 1 ou 5,
caractérisé en ce que la roue de roulement (21), pour la génération du second mouvement, sous l'action de la languette (203), repose de façon alternative sur la première voie à cames (202) et est relevée d'un espacement d'air (x) de la seconde voie à cames (201).

7. Dispositif selon l'une des revendications 1 ou 5,
caractérisé en ce que la roue de roulement (21) est relevée de la seconde voie à cames (201) de l'espacement d'air (x) lorsque le second mouvement est découplé du premier mouvement.
